# EUROPEAN PATENT APPLICATION

(11) **EP 3 753 924 A1**
(43) Date of publication of application: **23.12.2020**
(21) Application number: 19180740.3
(22) Date of filing: 18.06.2019
(51) Int. Cl.: C07D 209/60, A61P 9/00, A61P 25/04, A61P 29/00, A61P 35/00, A61K 31/403

(54) **NEW TRICYCLIC 5-HT2 ANTAGONISTS**

(71) Applicant: AnaMar AB, 111 36 Stockholm (SE)
(72) Inventor: Pettersson, Lars, 224 80 LUND (SE)
(74) Representative: Groth & Co. KB

(57) **Abstract**

The present invention relates to tricyclic 1-amidino-4-methyl-[2,3-fused]-2-pyrroline derivatives of the general formula I

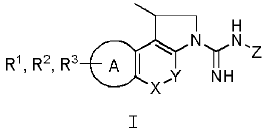

The invention specifically relates to such derivatives which exhibit antagonizing activity towards serotonin 5-HT_{2B} receptors. The present invention also relates to use of said compounds as a medicament and for the treatment of fibrosis, cardiovascular diseases, pain, IBD, inflammatory diseases, and cancer, as well as pharmaceutical compositions comprising one or more of said compounds and methods of treatment.

## Description

### Field of the invention

The present invention relates to novel 5-HT_{2B} receptor antagonists. The invention specifically relates to such derivatives which exhibit antagonizing activity towards serotonin 5-HT_{2B} receptors. The present invention also relates to use of said compounds as a medicament and for the treatment of fibrosis, cardiovascular diseases, pain, IBD, inflammatory diseases, and cancer, as well as pharmaceutical compositions comprising one or more of said compounds and methods of treatment.

### Background of the invention

Serotonin (5-Hydroxytryptamine, 5-HT) is a well characterized neurotransmitter and vasoactive amine which has been implicated in common disorders involving central nervous, gastrointestinal, cardiovascular and pulmonary systems. Peripheral 5-HT is mainly synthesized and released by the enterochromaffin cells in the gut. When reaching the blood stream it is sequestered inside platelets. Under normal conditions the level of free 5-HT in plasma is low and strictly regulated by specific 5-HT transporters present on the surface of e.g. platelets as well as by 5-HT degrading enzymes. Upon activation platelets release 5-HT and a local increase in 5-HT concentration is observed. Over the years evidence has gathered that 5-HT has a significant role in the functioning of the mammalian body. For example, it has been shown to regulate processes like cardiovascular function, bowel motility and bladder control. 5-HT and the 5-HT receptor system have also been associated with the modulation of pain and more specifically, the 5-HT₂ receptors have been shown to play an important role in the inflammatory pain process.

A greater understanding of 5-HT function has emerged with the characterization of its, at least, 14 different human receptors which are grouped into subfamilies based on their structural and pharmacological differences. Each receptor exhibits unique distribution and shows various preference for different ligands. The receptors are all G protein-coupled receptors, except for the 5-HT₃ receptor, which is a ligand-gated ion channel. The 5-HT₂ receptor family consists of 3 subtypes, 5-HT_{2A}, 5-HT_{2B} and 5-HT_{2C}. The 5-HT₂ receptors share significant sequence homology at the amino acid level and couple to the Gq family of the G proteins.

The 5-HT_{2B} receptor is expressed in inflammatory cells and the receptor is involved in 5-HT induced production of IL-1β, IL-6 and TNF-α in mouse cardiac fibroblasts. In the LPS-induced inflammatory mouse model reduction of TNF-α levels has been shown after treatment with a selective 5-HT_{2B} receptor antagonist (WO2016/207231A1).

The 5-HT_{2B} receptor has previously been linked to pulmonary arterial hypertension (PAH) and the phenotype of the 5-HT_{2B} receptor knock-out mice shows its importance for heart development. It demonstrates that 5-HT via the 5-HT_{2B} receptor regulates differentiation and proliferation of developing and adult heart. Conversely, over-expression of 5-HT_{2B} in mice leads to cardiac hypertrophy.

In agreement with this, 5-HT and its receptors, 5-HT_{2A} and 5-HT_{2B} in particular, have been implicated in the etiology of several fibrotic disorders including retroperitoneal fibrosis, carcinoid heart disease, scleroderma, liver and lung fibrosis. Fibrosis is actually a feature of many different types of chronic respiratory diseases including IPF, PAH, COPD and asthma. A mechanistic link between fibrosis and 5-HT was first reported in the 1960s for a condition called carcinoid syndrome which is caused by neuroendocrine carcinoid tumours that secrete vast quantities of 5-HT. The syndrome is characterized by tissue fibrosis that particularly affects cardiac valves but also impacts on other organs including lung and skin. Subsequently, agonism on the 5-HT_{2B} receptor has been implicated in fibrosis caused by fenfluramine used in the treatment of obesity and psychiatric disorders. Fibrosis is characterized by enhanced fibroblast/myofibroblast proliferation and activation which results in an altered extracellular matrix deposition which ultimately results in organ failure.

An important mediator of the fibrotic process is transforming growth factor beta, TGF-β. This cytokine modulates a variety of physiological processes through transcriptional regulation. In human lung fibroblasts, TGF-β is well-known for inducing myofibroblast differentiation with increased expression levels of alpha-SMA in intracellular stress fibers as well as an increased matrix deposition.

A lot of evidence supports a role of 5-HT in fibrosis although the exact mechanism how 5-HT promotes fibrosis is not defined. 5-HT has been shown to increase the production of TGF-β via the 5-HT_{2B} receptor and in models of scleroderma human dermal fibroblasts have a dose-dependent increase of TGF-β mRNA in response to 5-HT as well as an increased expression of the 5-HT_{2B} receptor. This results in an increased expression of collagen 1a1, collagen 1a2 and fibronectin. The effects of 5-HT on matrix synthesis were blocked by a 5-HT_{2B} receptor antagonist or by transfected 5-HT_{2B} siRNAs. The same study showed that selective 5-HT_{2B} receptor antagonists prevent bleomycin-induced dermal fibrosis in vivo (Dees C. et al., J. Exp. Med. (2011) 208(5), 961-72). In other fibrotic diseases such as liver fibrosis, treatment with 5-HT_{2B} receptor antagonists resulted in attenuated fibrogenesis in an *in vivo* model of chronic liver disease (Ebrahimkhani, M.R. et al., Nat Med. (2011) 17(12), 1668-73). Further support for 5-HT and fibrosis is found in patients suffering from IPF that have an increased expression of 5-HT_{2A} and 5-HT_{2B} receptors in the fibrotic lung. Another study identified strong fibroblast expression of 5-HT_{2B} receptor, in fibroblastic foci in human lung samples from IPF patients. In addition, treatment with Terguride, a 5-HT_{2A} and 5-HT_{2B} receptor antagonist, reduce the expression of type I collagen in TGF-β1 stimulated human lung fibroblasts. In the bleomycin (BLM)-induced lung fibrosis model in mice, anti-fibrotic effects are seen after treatment with 5-HT_{2A} and 5-HT_{2B} receptor antagonists and also with a selective 5-HT_{2B} antagonist (WO2016/207231A1).

Wound healing, chronic fibrosis and tumors have similar characteristics where the presence of myofibroblasts has emerged as a common hallmark. Persistent myofibroblast populations are found during chronic tissue fibrosis and in tumor-activated stroma (desmoplasia) while they are transiently present during acute wound healing. The myofibroblasts produce excessive amounts of extracellular matrix (ECM) and in cancer this results in formation of a tumor stroma with critical roles in cancer development, progression and metastasis. The tumor stroma has a dense and altered ECM and represents a challenge in treatment due to a severely impaired access of drugs to the tumor. A more effective antitumor-drug delivery can therefore be achieved by anti-fibrotic drug treatment. Fibrosis has also been shown to constitute a pre-disposition for certain carcinomas such as pancreatic and hepatic cell carcinomas (Rybinski, B. et al., Physiol Genomics (2014) 46(7), 223-44).
In addition to the suggested involvement in tumor stroma, 5-HT has more recently emerged as a growth factor for human tumor cells. 5-HT production and secretion by neuroendocrine cells have been shown in the progression of several solid tumors. A recent study showed that levels of 5-HT were increased in human pancreatic ductal adenocarcinoma (PDAC) tissues compared with non-tumor pancreatic tissues. 5-HT increased proliferation in a PDAC cell line and prevented apoptosis. The 5-HT_{2B} receptor showed an increased expression in PDAC and agonists to the 5-HT_{2B} receptor, but no other 5-HT receptors, promoted proliferation and prevented apoptosis in PDAC cells while 5-HT_{2B} receptor inhibitors reduced their growth as xenograft tumors in mice (Jiang, S.H. et al., Gastroenterology (2017) 153(1), 277-91 e19). The 5-HT_{2B} receptor is also present in human hepatocellular carcinoma (HCC) and 5-HT promotes cell survival and growth of hepatocellular cancer cells by activation of the 5-HT_{2B} receptor. Furthermore, the 5-HT_{2B} receptor is involved in uveal melanoma (UM), where high levels of HTR2B mRNA transcript is a discriminating mark to distinguish metastatic and non-metastatic UM cell lines. Pharmacological inhibition with a 5-HT_{2B} receptor antagonist in UM cells reduced viability and impaired potential of migration (Weidmann, C. et al., Clin Exp Metastasis (2018) 35(3), 123-34).

### 5-HT_{2B} antagonists

Many 5-HT_{2B} antagonists of variable structural classes have been described in the literature; for reviews, see Poissonnet, G., et al., Mini-Reviews in Medicinal Chemistry (2004), 4(3), 325-330 and Brea, J. et al., Current Topics in Medicinal Chemistry (Sharjah, United Arab Emirates) (2010), 10(5), 493-503. These include the di-ureas SB206553 and SB215505, the piperazine derivative EGIS-7625, the 2-amino-4-naphthyl-pyrimidine MT-500 (RS127445), thioxanthene structures, the ergot derivative terguride, tetrahydro-β-carbolines, the thienopyrimidine PRX-08066, and quinoline derivatives. Other examples of 5-HT_{2B} antagonists are disclosed in US8252790B2, US2009062363A1, EP1728784A1, and WO2016/207231A1.
Still there remains a need to develop new potent and selective 5-HT_{2B} antagonists for drug development.

### Summary of the invention

It is an object of the present invention to provide new antagonists of the serotonin 5-HT_{2B} receptors for the treatment of diseases, such as fibrosis, cardiovascular diseases, pain, IBD, inflammatory diseases, and cancer.

The present invention relates to a compound of the general formula I

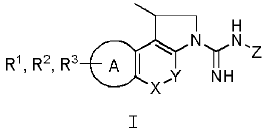

It has been found that 1-amidino-4-methyl-[2,3-fused]-2-pyrroline derivatives, as specified in the appended claims, are 5-HT_{2B} receptor antagonists with high potency and/or selectivity. In particular, the amidated rigid methylated fused tricyclic structure of the compounds of the present invention has been identified as a useful backbone for 5-HT_{2B} receptor antagonists.

### Detailed description of the invention

In one aspect of the invention, there is provided a compound of the general formula I 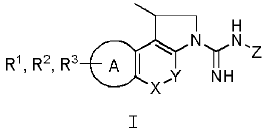 wherein
ring A 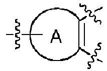 represents a 5-6 membered aromatic or heteroaromatic ring containing 0-3 heteroatoms independently selected from N, O, and S;
R¹, R², and R³, are independently selected from hydrogen, methyl, ethyl, n-propyl, iso-propyl, cyclopropyl, tert-Bu, ethynyl, CF₃, hydroxy, methoxy, ethoxy, iso-propoxy, OCF₃, SCH₃, S(O)₂OH, S(O)CH₃, S(O)₂CH₃. S(O)₂NH₂, S(O)₂N(CH₃)₂, NH₂, NHCH₃, N(CH₃)₂, NHC(O)CH₃, C(O)N(CH₃)₂, F, Cl, Br, I, CN, and 5-6 membered aromatic or heteroaromatic rings containing 0-3 heteroatoms independently selected from N, O, and S; X-Y, in which X is connected to Y by a single or a double bond or is non-existing, is selected from CH=CH, C(CH₃)=CH, C(F)=CH, C(Cl)=CH, C(OMe)=CH, CH₂-CH₂, N=CH, CH=N, N=N, O-CH₂, O-C(O), NH, NCH₃, O, or S;
Z is selected from hydrogen, methyl, ethyl, n-propyl, iso-propyl, n-butyl, iso-butyl, neo-butyl, tert-butyl, allyl, 2-propynyl, cyclopropyl, cyclopropylmethyl, cyclopentyl, cyclohexyl, CH₂CCH, CF₃, CH₂CF₃, CH₂CH₂F, CH₂CH₂CF₂CF₃, phenyl, benzyl, hydroxy, and methoxy, wherein said phenyl and benzyl groups are optionally mono- or di-substituted by substituents independently selected from methyl, methoxy, F, Cl, and CF₃; and
pharmaceutically acceptable salts, prodrugs, tautomers, and stereoisomers thereof.

X-Y, in which X is connected to Y by a single or a double bond or is non-existing, is selected from CH=CH, C(CH₃)=CH, C(F)=CH, C(Cl)=CH, C(OMe)=CH, CH₂-CH₂, N=CH, CH=N, N=N, O-CH₂, O-C(O), NH, NCH₃, O, or S. In other words, when X is connected to Y by a single bond, X-Y is selected from CH₂-CH₂, O-CH₂, or O-C(O). When X is connected to Y by a double bond, X-Y is selected from CH=CH, C(CH₃)=CH, C(F)=CH, C(Cl)=CH, C(OMe)=CH, N=CH, CH=N, or N=N. When X is non-existing, X-Y is selected from NH, NCH₃, O, or S.

In some embodiments 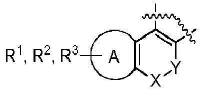 is selected from the group 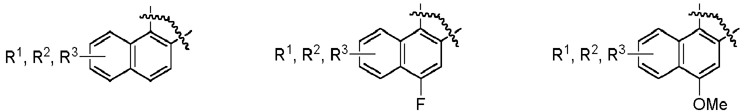 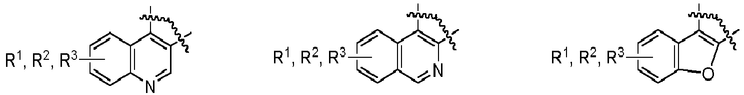 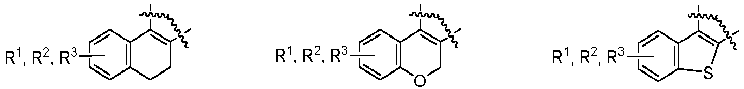 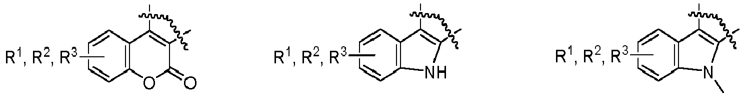 wherein
R¹, R², and R³, are independently selected from hydrogen, methyl, ethyl, n-propyl, iso-propyl, cyclopropyl, tert-Bu, ethynyl, CF₃, hydroxy, methoxy, ethoxy, iso-propoxy, OCF₃, SCH₃, S(O)₂OH, S(O)CH₃, S(O)₂CH₃. S(O)₂NH₂, S(O)₂N(CH₃)₂, NH₂, NHCH₃, N(CH₃)₂, NHC(O)CH₃, C(O)N(CH₃)₂, F, Cl, Br, I, CN, and 5-6 membered aromatic or heteroaromatic rings containing 0-3 heteroatoms independently selected from N, O, and S.

In some embodiments 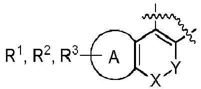 is selected from the group 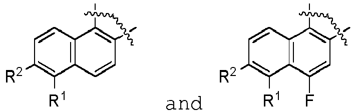 wherein
R¹ is selected from hydrogen, methyl, ethyl, iso-propyl, cyclopropyl, tert-Bu, ethynyl, CF₃, methoxy, OCF₃, SCH₃, S(O)₂NH₂, S(O)₂N(CH₃)₂, F, Cl, Br, I, CN, phenyl, thiophen-2-yl, thiophen-3-yl, pyrazol-3-yl, pyrazol-4-yl, imidazole-2-yl, imidazole-4-yl, isoxazole-3-yl, isoxazole-4-yl; and
R² is selected from hydrogen and F.

In some embodiments, Z is selected from hydrogen, methyl, ethyl, n-propyl, iso-propyl, n-butyl, cyclopropyl, allyl, 2-propynyl, CH₂CH₂F, CH₂CF₃, CH₂CH₂CF₂CF₃, phenyl, and benzyl, wherein said phenyl and benzyl groups are optionally mono- or di-substituted by substituents independently selected from methyl, methoxy, F, Cl, and CF₃.

In some embodiments 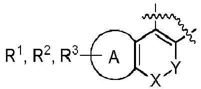 is selected from the group 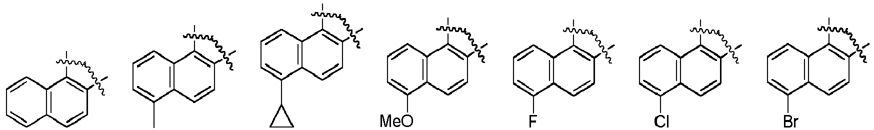 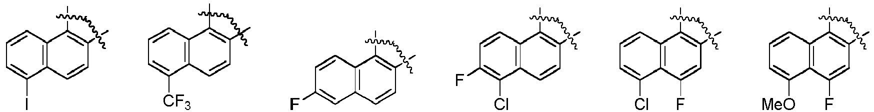 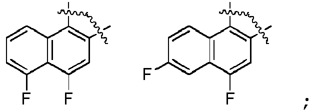 and
Z is selected from hydrogen, methyl, n-butyl, cyclopropyl, allyl, 2-propynyl, CH₂CH₂F, CH₂CF₃, CH₂CH₂CF₂CF₃, phenyl, benzyl, wherein said phenyl and benzyl groups are optionally mono- or di-substituted by substituents independently selected from methyl, methoxy, F, Cl, and CF₃.

In some embodiments ring A 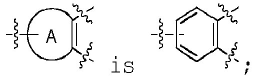
R¹, R², and R³, are independently selected from hydrogen, F, and Br;
X-Y is selected from CH=CH, N=CH, and CH=N; and
Z is selected from hydrogen, methyl, n-butyl, and benzyl, wherein said benzyl is optionally mono- or di-substituted by substituents independently selected from F and Cl.

In some embodiments, the absolute configuration is (S) as shown in formula Ia

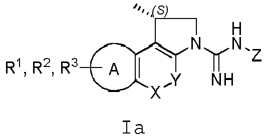

In some embodiments, the compound is selected from:
1-Methyl-1H-benzo[e]indole-3(2H)-carboximidamide;
N,1-Dimethyl-1H-benzo[e]indole-3(2H)-carboximidamide;
N-Butyl-1-methyl-1H-benzo[e]indole-3(2H)-carboximidamide;
N-Allyl-1-methyl-1,2-dihydro-3H-benzo[e]indole-3-carboximidamide;
1-Methyl-N-(prop-2-yn-1-yl)-1,2-dihydro-3H-benzo[e]indole-3-carboximidamide;
N-(2-Fluoroethyl)-1-methyl-1,2-dihydro-3H-benzo[e]indole-3-carboximidamide;
N-Benzyl-1-methyl-1H-benzo[e]indole-3(2H)-carboximidamide N-(4-Fluorobenzyl)-1-methyl-1,2-dihydro-3H-benzo[e]indole-3-carboximidamide;
N-(3,4-Difluorobenzyl)-1-methyl-1H-benzo[e]indole-3(2H)-carboximidamide;
N-(4-Chlorobenzyl)-1-methyl-1H-benzo[e]indole-3(2H)-carboximidamide;
1-Methyl-N-(4-(trifluoromethyl)benzyl)-1,2-dihydro-3H-benzo[e]indole-3-carboximidamide;
N-(4-Methoxybenzyl)-1-methyl-1,2-dihydro-3H-benzo[e]indole-3-carboximidamide;
1-Methyl-N-phenyl-1,2-dihydro-3H-benzo[e]indole-3-carboximidamide;
N-(4-Fluorophenyl)-1-methyl-1,2-dihydro-3H-benzo[e]indole-3-carboximidamide;
N-(4-Chlorophenyl)-1-methyl-1,2-dihydro-3H-benzo[e]indole-3-carboximidamide;
1-Methyl-N-(4-(trifluoromethyl)phenyl)-1,2-dihydro-3H-benzo[e]indole-3-carboximidamide;
N-(4-Methoxyphenyl)-1-methyl-1,2-dihydro-3H-benzo[e]indole-3-carboximidamide;
6-Fluoro-1-methyl-1,2-dihydro-3H-benzo[e]indole-3-carboximidamide;
6-Chloro-1-methyl-1,2-dihydro-3H-benzo[e]indole-3-carboximidamide;
6-Chloro-N,1-dimethyl-1,2-dihydro-3H-benzo[e]indole-3-carboximidamide;
N-Butyl-6-chloro-1-methyl-1,2-dihydro-3H-benzo[e]indole-3-carboximidamide;
N-Allyl-6-chloro-1-methyl-1,2-dihydro-3H-benzo[e]indole-3-carboximidamide;
6-Chloro-1-methyl-N-(prop-2-yn-1-yl)-1,2-dihydro-3H-benzo[e]indole-3-carboximidamide;
6-Chloro-N-(2-fluoroethyl)-1-methyl-1,2-dihydro-3H-benzo[e]indole-3-carboximidamide;
N-Benzyl-6-chloro-1-methyl-1,2-dihydro-3H-benzo[e]indole-3-carboximidamide;
6-Chloro-1-methyl-N-phenyl-1,2-dihydro-3H-benzo[e]indole-3-carboximidamide;
6-Bromo-1-methyl-1H-benzo[e]indole-3(2H)-carboximidamide;
6-Bromo-N,1-dimethyl-1,2-dihydro-3H-benzo[e]indole-3-carboximidamide;
6-Bromo-N-butyl-1-methyl-1,2-dihydro-3H-benzo[e]indole-3-carboximidamide;
N-Allyl-6-bromo-1-methyl-1,2-dihydro-3H-benzo[e]indole-3-carboximidamide;
6-Bromo-1-methyl-N-(prop-2-yn-1-yl)-1,2-dihydro-3H-benzo[e]indole-3-carboximidamide;
6-Bromo-N-(2-fluoroethyl)-1-methyl-1,2-dihydro-3H-benzo[e]indole-3-carboximidamide;
N-Benzyl-6-bromo-1-methyl-1,2-dihydro-3H-benzo[e]indole-3-carboximidamide;
6-Bromo-1-methyl-N-phenyl-1,2-dihydro-3H-benzo[e]indole-3-carboximidamide;
6-Iodo-1-methyl-1,2-dihydro-3H-benzo[e]indole-3-carboximidamide;
1,6-Dimethyl-1,2-dihydro-3H-benzo[e]indole-3-carboximidamide;
6-Cyclopropyl-1-methyl-1,2-dihydro-3H-benzo[e]indole-3-carboximidamide;
1-Methyl-6-(trifluoromethyl)-1,2-dihydro-3H-benzo[e]indole-3-carboximidamide;
N,1-Dimethyl-6-(trifluoromethyl)-1,2-dihydro-3H-benzo[e]indole-3-carboximidamide;
N-Butyl-1-methyl-6-(trifluoromethyl)-1,2-dihydro-3H-benzo[e]indole-3-carboximidamide;
N-Allyl-1-methyl-6-(trifluoromethyl)-1,2-dihydro-3H-benzo[e]indole-3-carboximidamide;
1-Methyl-N-(prop-2-yn-1-yl)-6-(trifluoromethyl)-1,2-dihydro-3H-benzo[e]indole-3-carboximidamide;
N-(2-Fluoroethyl)-1-methyl-6-(trifluoromethyl)-1,2-dihydro-3H-benzo[e]indole-3-carboximidamide;
N-Benzyl-1-methyl-6-(trifluoromethyl)-1,2-dihydro-3H-benzo[e]indole-3-carboximidamide;
1-Methyl-N-phenyl-6-(trifluoromethyl)-1,2-dihydro-3H-benzo[e]indole-3-carboximidamide;
6-Methoxy-1-methyl-1,2-dihydro-3H-benzo[e]indole-3-carboximidamide;
6-Chloro-7-fluoro-1-methyl-1,2-dihydro-3H-benzo[e]indole-3-carboximidamide;
6-Chloro-5-fluoro-1-methyl-1,2-dihydro-3H-benzo[e]indole-3-carboximidamide;
5-Fluoro-6-methoxy-1-methyl-1,2-dihydro-3H-benzo[e]indole-3-carboximidamide;
5,6-Difluoro-1-methyl-1,2-dihydro-3H-benzo[e]indole-3-carboximidamide;
7-Fluoro-1-methyl-1H-benzo[e]indole-3(2H)-carboximidamide;
7-Fluoro-N,1-dimethyl-1,2-dihydro-3H-benzo[e]indole-3-carboximidamide;
N-Butyl-7-fluoro-1-methyl-1,2-dihydro-3H-benzo[e]indole-3-carboximidamide;
N-Allyl-7-fluoro-1-methyl-1,2-dihydro-3H-benzo[e]indole-3-carboximidamide;
7-Fluoro-1-methyl-N-(prop-2-yn-1-yl)-1,2-dihydro-3H-benzo[e]indole-3-carboximidamide;
7-Fluoro-N-(2-fluoroethyl)-1-methyl-1,2-dihydro-3H-benzo[e]indole-3-carboximidamide;
N-Benzyl-7-fluoro-1-methyl-1,2-dihydro-3H-benzo[e]indole-3-carboximidamide;
7-Fluoro-1-methyl-N-phenyl-1,2-dihydro-3H-benzo[e]indole-3-carboximidamide;
5,7-Difluoro-1-methyl-1,2-dihydro-3H-benzo[e]indole-3-carboximidamide;
1-Methyl-1H-pyrrolo[2,3-c]isoquinoline-3(2H)-carboximidamide; and 1-Methyl-1H-pyrrolo[2,3-c]quinoline-3(2H)-carboximidamide.

In some embodiments, the compound is selected from:
7-Fluoro-1-methyl-1H-benzo[e]indole-3(2H)-carboximidamide;
6-Bromo-1-methyl-1H-benzo[e]indole-3(2H)-carboximidamide;
1-Methyl-1H-benzo[e]indole-3(2H)-carboximidamide;
1-Methyl-1H-pyrrolo[2,3-c]isoquinoline-3(2H)-carboximidamide;
1-Methyl-1H-pyrrolo[2,3-c]quinoline-3(2H)-carboximidamide;
N,1-Dimethyl-1H-benzo[e]indole-3(2H)-carboximidamide;
N-Butyl-1-methyl-1H-benzo[e]indole-3(2H)-carboximidamide;
N-Benzyl-1-methyl-1H-benzo[e]indole-3(2H)-carboximidamide;
N-(4-Chlorobenzyl)-1-methyl-1H-benzo[e]indole-3(2H)-carboximidamide; and
N-(3,4-Difluorobenzyl)-1-methyl-1H-benzo[e]indole-3(2H)-carboximidamide.

In another aspect of the invention there is provided a compound of formula I for use as a medicament.

In another aspect of the invention there is provided a compound of formula I, for use in treatment of fibrosis, cardiovascular diseases, pain, IBD, inflammatory diseases, or cancer. Typically, fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring. Typically, said cardiovascular disease is selected from atherosclerosis and hypertension. Typically, said pain is selected from migraine and pain associated with inflammatory diseases. Typically, said IBD is selected from Crohn's disease and ulcerous colitis. Typically, said cancer is selected from extracellular matrix producing cancers such as breast cancer, pancreas cancer, and liver cancer.

In another aspect of the invention there is provided a compound of formula I, for use in treatment of inflammatory joint diseases including rheumatoid arthritis (RA) and osteoarthritis (OA).

In another aspect of the invention there is provided use of a compound of formula I, in the manufacture of a medicament useful in treatment of fibrosis, cardiovascular diseases, pain, IBD inflammatory diseases, or cancer. Typically, said fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring. Typically, said cardiovascular disease is selected from atherosclerosis and hypertension. Typically, said pain is selected from migraine and pain associated with inflammatory diseases. Typically, said IBD is selected from Crohn's disease and ulcerous colitis. Typically, said cancer is selected from extracellular matrix producing cancers such as breast cancer, pancreas cancer, and liver cancer.

In another aspect of the invention there is provided use of a compound of formula I, in the manufacture of a medicament useful in treatment of inflammatory joint diseases including rheumatoid arthritis (RA) and osteoarthritis (OA).

In another aspect of the invention there is provided a method of treating fibrosis, cardiovascular diseases, pain, IB, inflammatory diseases, or cancer comprising administering a therapeutically effective amount of a compound of Formula I to a patient in need thereof. Typically, said fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring. Typically, said cardiovascular diseases are selected from atherosclerosis and hypertension. Typically, said pain is selected from migraine and pain associated with inflammatory diseases. Typically, sais IBD is selected from Crohn's disease and ulcerous colitis. Typically, said cancer is selected from extracellular matrix producing cancers such as breast cancer, pancreas cancer, and liver cancer.

In another aspect of the invention there is provided a method of treating inflammatory joint diseases including rheumatoid arthritis (RA) and osteoarthritis (OA), comprising administering a therapeutically effective amount of a compound of formula I to a patient in need thereof.

In another aspect of the invention there is provided a pharmaceutical composition comprising a compound according to Formula I, admixed with one or more pharmaceutically acceptable excipients or carriers. Typically, said excipients are selected from the group comprising filling agents, lubricants, flavours, colourings, sweetenings, buffers, acidifying agents, diluents, and preservatives. Typically, said compositions are formulated to be administered orally, by oral inhalation, intramuscularly, intravenously, intraperitoneally, or subcutaneously, via implants, rectally, intranasally, or transdermally; preferably orally.

The compounds of the invention may be used in the prophylaxis and treatment as such, or preferably in a form of a pharmaceutical composition. While it is possible for the active ingredient to be administered alone, it is preferable for it to be present in a pharmaceutical formulation or composition. Accordingly, the invention provides a pharmaceutical formulation comprising a compound according to the invention, and a pharmaceutically acceptable diluent, excipient or carrier (collectively referred to herein as "carrier" materials). Pharmaceutical compositions of the invention may take the form of a pharmaceutical formulation as described below. Thus, the present invention relates to a pharmaceutical composition containing at least one compound of formula I together with conventional excipients.

Exemplary compositions for oral administration include suspensions (including nanosuspensions) which can contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which can contain, for example, microcrystalline cellulose, dicalcium phosphate, starch, magnesium stearate, calcium sulfate, sorbitol, glucose, and/or lactose and/or other excipients, binders, extenders, disintegrants, diluents, and lubricants such as those known in the art. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes, and the like. Disintegrators include without limitation starch, methylcellulose, agar, bentonite, xanthan gum, and the like. The compounds of formula I can also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose, and/or cyclodextrins. Also included in such formulations may be high molecular weight excipients such as celluloses (avicel) or polyethylene glycols (PEG). Such formulations can also include an excipient to aid mucosal adhesion such as hydroxy propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g. Carbopol 934). Lubricants, glidants, flavors, coloring agents, and stabilizers may also be added for ease of fabrication and use. Lubricants used in these dosage forms include sodium oleate, sodium stearate, magnesium stearate, sodium benzoate, sodium acetate, sodium chloride, and the like. For oral administration in liquid form, the oral drug components can be combined with any oral, non-toxic, pharmaceutically acceptable inert carrier such as ethanol, glycerol, water, and the like.

The pharmaceutical formulations according to the invention include those suitable for oral, parenteral [including subcutaneous, intradermal, intramuscular, intravenous (bolus or infusion), and intraarticular], inhalation (including fine particle dusts or mists which may be generated by means of various types of metered dose pressurized aerosols), nebulizers or insufflators, rectal, intraperitoneal, and topical (including dermal, buccal, sublingual, and intraocular) administration, although the most suitable route may depend upon, for example, the condition and disorder of the recipient.

Formulations of the present invention suitable for oral administration may be presented as discrete units such as capsules, cachets, pills or tablets each containing a predetermined amount of the active ingredient; as a powder or granules; as a solution or a suspension in an aqueous liquid or a non-aqueous liquid, for example as elixirs, tinctures, suspensions (including nanosuspensions) or syrups; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The present compounds can, for example, be administered in a form suitable for immediate release or extended release. Immediate release or extended release can be achieved by the use of suitable pharmaceutical compositions comprising the present compounds, or, particularly in the case of extended release, by the use of devices such as subcutaneous implants or osmotic pumps. The present compounds can also be administered liposomally. Preferred unit dosage formulations are those containing an effective dose, as hereinbefore recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The formulations may conveniently be presented in unit dosage form and may be prepared by any of the methods well known in the art of pharmacy. All methods include the step of bringing the active ingredient into association with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

The compounds of the present invention can also be administered in the form of liposome delivery systems, such as small unilamellar vesicles, large unilamellar vesicles, and multilamellar vesicles. Liposomes can be formed from a variety of phospholipids, 1,2-dipalmitoylphosphatidylcholine, phosphatidyl ethanolamine (cephaline), phosphatidylserine, phosphatidylinositol, diphosphatidylglycerol (cardiolipin) or phosphatidylcholine (lecithin).

Formulations for parenteral administration include aqueous and non-aqueous sterile injection solutions which may contain anti-oxidants, buffers, bacteriostats, and solutes which render the formulation isotonic with the blood of the intended recipient; and aqueous and non-aqueous sterile suspensions (including nanosuspensions) which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example saline or water-for-injection, immediately prior to use. Extemporaneous injection solutions and suspensions may be prepared from sterile powders, granules, and tablets of the kind previously described. Exemplary compositions for parenteral administration include injectable solutions or suspensions which can contain, for example, suitable non-toxic, parenterally acceptable diluents or solvents, such as polyethylene glycol, ethanol, 1,3-butanediol, water, Ringer's solution, an isotonic sodium chloride solution, or other suitable dispersing or wetting and suspending agents, including synthetic mono- or diglycerides, and fatty acids, including oleic acid, polysorbates, and Cremaphor.

Exemplary compositions for nasal, aerosol or inhalation administration include solutions in saline, which can contain, for example, benzyl alcohol or other suitable preservatives, absorption promoters to enhance bioavailability, and/or other solubilizing or dispersing agents such as those known in the art.

Formulations for rectal administration may be presented as a suppository with the usual carriers such as cocoa butter, synthetic glyceride esters or polyethylene glycol. Such carriers are typically solid at ordinary temperatures, but liquefy and/or dissolve in the rectal cavity to release the drug.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerine or sucrose and acacia. Exemplary compositions for topical administration include a topical carrier such as Plastibase (mineral oil gelled with polyethylene).

The amount of active ingredient which is required to achieve a therapeutic effect will, of course, vary with the particular compound, the route of administration, the subject under treatment, including the type, species, age, weight, sex, and medical condition of the subject and the renal and hepatic function of the subject, and the particular disorder or disease being treated, as well as its severity. An ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 mg per kg of body weight per day (mg/kg/day) to 10 mg/kg/day, and most preferably 0.1 to 5.0 mg/kg/day, for adult humans. For oral administration, the compositions are preferably provided in the form of tablets or other forms of presentation provided in discrete units containing 0.01, 0.05, 0.1, 0.5, 1.0, 2.5, 5.0, 10.0, 15.0, 25.0, 50.0, 100, and 500 milligrams of the active ingredient for the symptomatic adjustment of the dosage to the patient to be treated. A medicament typically contains from about 0.01 mg to about 500 mg of the active ingredient. Furthermore, preferred compounds for the present invention can be administered in intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen.

In some embodiments, the composition further comprises an additional therapeutic agent.

Compounds of general formula (I) may be administered as the sole pharmaceutical agent or in combination with one or more additional therapeutic agents where the combination causes no unacceptable adverse effects. This pharmaceutical combination includes administration of a single pharmaceutical dosage formulation which contains a compound of general formula (I) and one or more additional therapeutic agents, as well as administration of the compound of general formula (I) and each additional therapeutic agent in its own separate pharmaceutical dosage formulation. For example, a compound of general formula (I) and a therapeutic agent may be administered to the patient together in a single oral dosage composition such as a tablet or capsule, or each agent may be administered in separate dosage formulations.

Where separate dosage formulations are used, the compound of general formula (I) and one or more additional therapeutic agents may be administered at essentially the same time (e.g. concurrently) or at separately staggered times (e.g. sequentially).

In particular, the compounds of the present invention may be used in fixed or separate combination with effectors of nuclear receptors, transcription factors, G protein coupled receptors, ion channels, integrins, kinases, or enzymes.

In particular, the compounds of the present invention may be used in fixed or separate combination with: glucocorticoid receptor agonists e.g. triamcinolone, prednisone, prednisolone or budesonide; mineralocorticoid receptor antagonists e.g. spironolactone, eplerenone or canrenone; PPAR agonists e.g. rosiglitazone, GFT 505, saroglitazar, pioglitazone or farglitazar; FXR agonists e.g. obeticholic acid, Px 102 or ursodeoxycholic acid; PXR agonists e.g. pregnenolone 16α-carbonitrile; NR4A1 agonists e.g. cytosporone B; Nrf2 activators e.g. bardoxolone methyl; WNT / β-catenin inhibitors e.g. ICG-001; chemokine antagonists e.g. bindarit; LPA antagonists e.g. BMS 986020 or SAR 100842; prostacyclin analogues e.g. (+/-) beraprost sodium, iloprost or treprostinil; AT1 receptor antagonists e.g. losartan; ETA receptor antagonists e.g. atrasentan, ambrisentan, bosentan or macitentan; CCR5 antagonists e.g. maraviroc; CCR2 antagonists e.g. RS-504393; CXCR4 antagonists e.g. AMD3100; PAR1 inhibitors e.g. SCH 79797; S1P ligands e.g. fingolimod (FTY720); PTGER agonists e.g. (R)-rutaprost (prodrug); PTGFR antagonists e.g. AL-8810; LXA4 agonists e.g. BML-111; RXFP1 agonists; 5-HT2A or 5-HT2B receptor antagonists e.g. sarpogrelate; P2X7 antagonists e.g. A-438079; KCa3.1 / IKCa1 blockers e.g. TRAM-34; T-type Ca2+ Channel blockers e.g. efonidipine; Na-K-Cl cotransporter inhibitors e.g. torsemide; αVβ6 integrin inhibitors e.g. CWHM 12; αVβ1 integrin inhibitors e.g. c8; Galectin 3 antagonists e.g. TD139; TGF-β or p38 inhibitors e.g. pirfenidone or F-351; tyrosine kinase inhibitors e.g. nintedanib, imatinib or nilotinib; kinase inhibitors e.g. sorafenib, dasatinib, baricitinib or tanzisertib; PI3K-mTOR inhibitors e.g. GSK2126458; MK2 inhibitors e.g. MMI 0100; IGFII antagonists e.g. PXS 64 or PXS 25; PKCδ inhibitors e.g. rottlerin; p38 MAPK inhibitors e.g. SB239063 or FR-167653; RHO kinase inhibitors e.g. Y-27632; FAK inhibitors e.g. PF-562271; ALK5 inhibitors e.g. SB-431542; SMAD3 inhibitors e.g. SIS-3; TGFβ1 inhibiting peptides e.g. disitertide; PDE inhibitors e.g. pentoxifylline or CTP 499; PDE5 inhibitors e.g. sildenafil; NADPH oxidase inhibitors e.g. GKT 137831; TAFI inhibitors e.g. UK 396,082; cathepsin B inhibitors e.g. VBY 376; caspase inhibitors e.g. emricasan; LOXL2 inhibitors e.g. β-aminopropionitrile; TGM2 antagonists e.g. NTU281; prolyl hydroxylase inhibitors e.g. HOE 077 or pyridine-2,4-dicarboxylate; inhibitors of BMP1 or BMP1-like proteinases e.g. UK-421045; neutrophil elastases e.g. ONO-5046; EPRS inhibitors e.g. halofuginone; TNKS1 inhibitors e.g. XAV939; ACE inhibitors e.g. enalapril; ATX inhibitors e.g. GWJ-A-23; AT1 receptor antagonists e.g. losartan; 5LO inhibitors e.g. zileuton; HMG-CoA reductase inhibitors (statins) e.g. atorvastatin, fluvastatin, lovastatin, pitavastatin, pravastatin, rosuvastatin or simvastatin; PAI1 antagonists e.g. TM5275; FKBP12 binders e.g. sirolimus; S100A9 binders e.g. paquinimod; methyl transfer cofactor e.g. ademetionine; immunomodulatory compounds e.g. thalidomide or pomalidomide; mitochondria-targeted antioxidants e.g. mitoquinone; vitamin derivatives e.g. pyridoxamine or α-tocopherol; purine antagonists e.g. azathioprine; ROS scavengers or anti-oxidants e.g. N-acetylcysteine, alpha lipoic acid or α-tocopherol; microtubules disrupters e.g. colchicine; copper chelators e.g. D-penicillamine; alkylators e.g. cyclophosphamide; HSP47 expression inhibitors or BET inhibitors e.g. (+) JQ-1; or interferon γ-1b.

The novel compounds of the present invention can be prepared by known methods described in the literature. A general synthetic route to prepare compounds of the present invention may include the following steps:
- Preparation of a bicyclic aromatic precursor.
- Addition of substituents and structural moieties to enable a pyrroline cyclization reaction.
- Cyclization to form the fused pyrroline ring providing the tricyclic ring-system.
- Amidation of the pyrroline nitrogen to form the guanidine moiety.

It is understood that protecting groups (PGs) may be employed and that addition, deletion, or transformation of substituents may be part of the synthetic routes.
In the method descriptions below specific substituents and protecting group chemistry are omitted for clarity.

Amidation of the pyrroline nitrogen (Scheme 1) can be performed using a number of different methods and reagents, e.g. reactions with MeSC(NH)NHZ or its salts at elevated temperature in pyridine or with amidino-pyrazole, optionally with di-t-BOC or di-CBZ protection of the amidino group. The protected amidino group, e.g. di-t-BOC-amidino, can be alkylated by alkylating reagents to introduce Z-substituents, e.g. using the Mitsunobu reaction. Another amidation method is to use cyanamide reagents (NC-NHZ). Alternatively, multi-step reactions can be used, e.g. reaction with cyanogen bromide followed by H₂N-Z addition, or reaction with iso-thiocyanates (Z-N=C=S) or protected iso-thiocyanate (e.g. BzNCS, deprotection by basic hydrolysis), followed by S-methylation and reaction with an amine (H₂N-Z) to give the guanidine product.

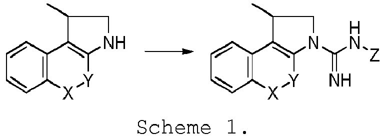

Cyclization to form the 3-methyl-pyrroline ring (Scheme 2) of the tricyclic derivatives can be performed by radical-induced cyclization of 2-allylamino-1-halo-naphthyl derivatives, typically with X = Br or I and with an N-protecting group, e.g. t-BOC (Tercel, M. et al., J Med Chem (2003) 46, 2132-51)

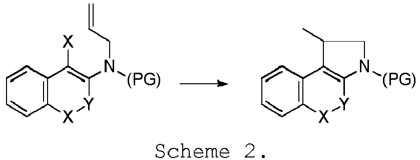

A similar method, described for 3-methylindolines, is to initiate cyclization by a carbolithiation reaction, which also allows for asymmetric induction by chiral ligands (Guyon, H. et al., J Org Chem (2017) 82, 4949-57).

An alternative cyclization method for β-tetralone derivatives is to alkylate the corresponding tetralone enamine with 2-bromopropionamide (Ghosh, D. et al., Eur J Med Chem (1995)30, 943-48) followed by reduction of the lactam carbonyl.

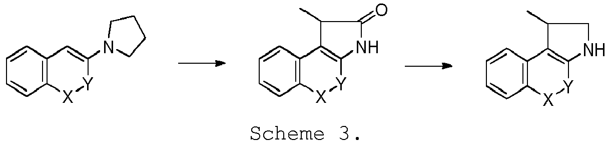

For the indole-pyrroline fused derivatives an oxidative cyclization method with e.g. t-BuOCl as oxidation reagent (Kawano, M. et al. Angewandte Chemie Int Ed (2013), 52(3), 906-10) can be used (Scheme 4).

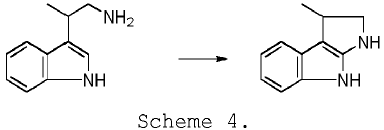

As shown above, the structures of the precursor compounds for the cyclization reactions, and the methods, can vary depending on the target compounds. However, a common theme is the ortho-allylamino halide substitution pattern as exemplified by (d) in Scheme 5 (halide exemplified with Br).

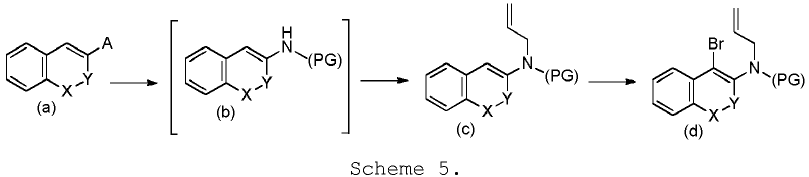

The preparation of amino derivatives (b) from the starting material (a) can be performed by many different methods: e.g. by reduction of a nitro group; by the Bucherer reaction; by the Ullmann- or Buchwald-type reactions; and by the Curtius-type rearrangement. Alternatively, the allylamino or protected amino derivatives (c) can be prepared directly from (a), e.g. by the Bucherer reaction, the Ullmann- or Buchwald-type reactions, or the Curtius-type rearrangement. Thus, the substituent A in starting material (a) can represent e.g. nitro, halide, hydroxyl, or carboxylic acid. In the case where the starting material (a) is an enol/keto compound, e.g. when Y is CH₂ and A is OH, this can be transformed to amino derivatives, including allylamino derivatives, by enamine formation.

The allyl moiety can also be introduced by allylation of the amine derivative (b) by reacting the amine with e.g. allyl-bromide or allylmesylate in presence of a base, e.g. triethylamine, or preferably NaH if the amine is protected by amide or carbamate protecting groups (PGs) such as t-BOC or CBZ.

The halogen, preferably Br or I, if not present in the starting material, can be introduced by halogenation reactions by reacting the amino or allylamino derivatives with halogenation reagents such as bromine, n-bromosuccinimide, or iodine.

In the preparative examples NMR analyses were performed on Varian Mercury or on Bruker UltraShield machines (frequencies and solvents as indicated). The chemical names of the copounds were generated using ChemBioDraw Ultra 12.0 (CambridgeSoft).

The present invention will now be described in more detail by the following examples, which are included in order to disclose certain embodiments of the invention, but not in any way to limit the scope of the invention.

### EXAMPLES

### Example 1

### 7-Fluoro-1-methyl-1H-benzo[e]indole-3(2H)-carboximidamide hydrochloride

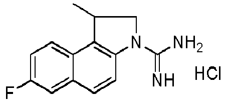

### Step 1: tert-Butyl (6-fluoronaphthalen-2-yl)carbamate

Diphenylphosphoryl azide (7.38 g, 26.8 mmol) was added to a solution of 6-fluoro-2-naphthoic acid (5.10 g, 26.8 mmol) and triethylamine (2.71 g, 26.8 mmol) in THF (85 mL). The reaction mixture was stirred at room temperature for 48 h and was then concentrated at reduced pressure. The residue was dissolved in t-BuOH (95 mL) and stirred at reflux temperature for 7 h. After cooling, the precipitated solid material was removed by filtration and the filtrate was concentrated at reduced pressure and the residue was partitioned in EtOAc (150 mL) and water (50 mL) basified with aq. NaOH (1 M, 25 mL). The organic phase was washed with water (2 x 50 mL), dried (Na₂SO₄), and concentrated at reduced pressure to give the title compound as beige crystals (4.43 g, 63%).
¹H NMR ((CD₃)₂SO, 400 MHz) δ: 1.50 (s, 9H), 7.34 (dt, 1H), 7.54 (dd, 1H), 7.58 (dd, 1H), 7.79 (d, 1H), 7.85 (dd, 1H), 8.14 (s, 1H), 9.58 (s, 1H).

### Step 2: 6-Fluoronaphthalen-2-amine

Trifluoroacetic acid (10 mL) was added to a solution of tert-butyl (6-fluoronaphthalen-2-yl)carbamate (4.35 g, 16.6 mmol) in CH₂Cl₂ (80 mL). The reaction mixture was stirred at room temperature for 20 h and was then concentrated at reduced pressure. The residue was partitioned in CH₂Cl₂ (100 mL) and water (75 mL) and basified with aq. KHCO₃ (sat.). The organic phase was washed with water (2 x 50 mL), dried (Na₂SO₄), and concentrated at reduced pressure to give the title compound as a solid material (2.65 g, 99%).
¹H NMR ((CD₃)₂SO, 400 MHz) δ: 5.35 (s, 2H), 6.86 (d, 1H), 6.99 (dd, 1H), 7.18 (dt, 1H), 7.41 (dd, 1H), 7.56 (dd, 1H), 7.58 (d, 1H).

### Step 3: 1-Bromo-6-fluoronaphthalen-2-amine

N-Bromosuccinimide (2.87 g, 16-1 mmol) was added in portions during 2 min to a solution of 6-fluoronaphthalen-2-amine (2.60 g, 16.1 mmol) in DMF (45 mL). After stirring the reaction mixture at room temperature for 20 h, water (100 mL) was slowly added and stirring was continued for 15 min. The precipitated crystals were collected by filtration, washed with water (2 x 50 mL) and dried to give the title compound as brown crystals (3.60 g, 93%).
¹H NMR ((CD₃)₂SO, 400 MHz) δ: 5.73 (s, 2H), 7.17 (d, 1H), 7.38 (dt, 1H), 7.56 (dd, 1H), 7.65 (d, 1H), 7.87 (dd, 1H).

### Step 4: tert-Butyl (1-bromo-6-fluoronaphthalen-2-yl)carbamate

Di-tert-butyl dicarbonate (9.76 g, 44.7 mmol) was added to a solution of 1-bromo-6-fluoronaphthalen-2-amine 3.58 g, 14.9 mmol), triethylamaine (1.81 g, 17.9 mmol) and 4-dimethylaminopyridine (0.18 g, 1.5 mmol) in CH₂Cl₂ (75 mL). The reaction mixture was stirred in a sealed vessel at 55-60°C for 48 h, cooled to room temperature and concentrated at reduced pressure. The residue was dissolved in MeOH (85 mL), K2CO3 (6.3 g) was added, and the reaction mixture was stirred at reflux temperature for 3 h. After cooling, water (125 mL) was added and stirring was continued for 15 min. The precipitated crystals were collected by filtration, washed with water (2 x 50 mL) and dried to give the title compound as brown crystals (4.8 g, 95%).
¹H NMR (CDCl₃, 400 MHz) δ: 1.58 (s, 9H), 7.26 (broad s, 1H), 7.34 (m, 1H), 7.43 (dd, 1H), 7.73 (d, 1H), 8.15 (dd, 1H), 8.37 (d, 1H).

### Step 5: tert-Butyl allyl(1-bromo-6-fluoronaphthalen-2-yl)carbamate

NaH (668 mg, 60% in oil, 16.7 mmol) was added in portions during 10 min to a solution of tert-butyl (1-bromo-6-fluoronaphthalen-2-yl)carbamate (4.74 g, 13.9 mmol) in dry THF (180 mL) at room temperature under argon. The reaction mixture was stirred for 15 min, DMF (25 mL) was added followed by allyl bromide (2.52 g, 20.8 mmol) and then the reaction mixture was stirred at room temperature for 24 h. MeOH (2 mL) was added to quench residual NaH, the reaction mixture was concentrated at reduced pressure, and the residue was partitioned in Et₂O (150 mL) and water (100 mL). The organic phase was washed with water (2 x 75 mL), dried (Na₂SO₄) and concentrated at reduced pressure. The residue was purified by silica column chromatography (heptane-EtOAc, 15:1) to give the title compound as an oil which solidified on standing (4.10 g, 78%).
¹H NMR (CDCl₃, 400 MHz) δ: 1.33, 1.56 (2s, rotamers, 9H), 3.97 (dd, 1H), 4.56 (dd, 1H), 5.04-5.15 (m, 2H), 5.96 (m, 1H), 7.29 (d, 1H), 7.37 (m, 1H), 7.47 (m, 1H), 7.71 (d, 1H), 8.15 (dd, 1H), 8.34 (dd, 1H) .

### Step 6: tert-Butyl 7-fluoro-1-methyl-1H-benzo[e]indole-3(2H)-carboxylate

Azobisisobutyronitrile (136 mg, 0.83 mmol) and tributyltinhydride (3.73 g, 12.8 mmol) was added to a solution of tert-butyl allyl(1-bromo-6-fluoronaphthalen-2-yl)carbamate (4.08 g, 10.7 mmol) in benzene (140 mL, degassed twice under argon) under argon. The reaction mixture was stirred at reflux temperature for 1.5 h, then cooled and concentrated at reduced pressure. The residue was purified by silica column chromatography (heptane-EtOAc, 15:1) to give the title compound as a pale yellow oil which solidified on standing (2.51 g, 78%).
¹H NMR (CDCl₃, 400 MHz) δ: 1.41 (d, 3H), 1.61 (broad s, 9H), 3.73-3.89 (m, 2H), 4.18 (dd, 1H), 7.26 (dt, 1H), 7.44 (dd, 1H), 7.66 (d, 1H), 7.74 (dd, 1H), 7.83, 8.23 (2 broad s, rotamers, 1H).

### Step 7: 7-Fluoro-1-methyl-2,3-dihydro-1H-benzo[e]indole

Trifluoroacetic acid (2.5 mL) was added to a solution of tert-butyl 7-fluoro-1-methyl-1H-benzo[e]indole-3(2H)-carboxylate (2.50 g, 8.29 mmol) in CH₂Cl₂ (25 mL). The reaction mixture was stirred at room temperature for 20 h and was then concentrated at reduced pressure. The residue was partitioned in CH₂Cl₂ (50 mL) and water (25 mL) and basified with aq. KHCO₃ (sat.) . The organic phase was washed with water (2 x 20 mL), dried (Na₂SO₄), and concentrated at reduced pressure to give the title compound as a brownish oil (1.60 g, 96%).
¹H NMR (CDCl₃, 400 MHz) δ: 1.39 (d, 3H), 3.37 (d, 1H), 3.75-3.89 (m, 3H), 7.04 (d, 1H), 7.22 (dt, 1H), 7.39 (dd, 1H), 7.54 (d, 1H), 7.67 (dd, 1H).

### Step 8: tert-Butyl (((tert-butoxycarbonyl)amino)(7-fluoro-1-methyl-1H-benzo[e]indol-3(2H)-yl)methylene)carbamate

N,N'-Bis-tert-butoxycarbonylpyrazole-1H-carboxamidine (632 mg, 2.04 mmol) was added to a solution of 7-fluoro-1-methyl-2,3-dihydro-1H-benzo[e]indole (410 mg, 2.04 mmol) in THF (12 mL) and the reaction mixture was stirred in a sealed vial at 70°C for 20 h. After cooling, the reaction mixture was concentrated at reduced pressure and the residue was purified by silica column chromatography (toluene-EtOAc, 50:1) to give the title compound as a foam (90 mg, 10%).
¹H NMR (CDCl₃, 400 MHz) δ: 1.44 (d, 3H), 1.48 (broad s, 18H), 3.72 (m, 1H), 4.01 (d, 1H), 4.44 (dd, 1H), 7.28 (dt, 1H), 7.43 (dd, 1H), 7.61 (d, 1H), 7.69 (broad s, 1H), 7.76 (dd, 1H), 10.44 (s, 1H).

### Step 9: 7-Fluoro-1-methyl-1H-benzo[e]indole-3(2H)-carboximidamide hydrochloride

Trifluoroacetic acid (0.4 mL) was added to a solution of tert-butyl (((tert-butoxycarbonyl)amino)(7-fluoro-1-methyl-1H-benzo[e]indol-3(2H)-yl)methylene)carbamate (90 g, 2.03 mmol) in CH₂Cl₂ (2 mL). The reaction mixture was stirred at room temperature for 20 h and was then concentrated at reduced pressure. The residue was dissolved in CH₂Cl₂ (2 mL), HCl/Et₂O (1 M, 1 mL) was added, and the solution was evaporated at reduced pressure to dryness. This procedure was repeated twice to remove the trifluoroacetic acid. The solid residue was stirred in Et₂O (3 mL), collected by filtration, washed with Et₂O and dried to give the title compound as pale greenish crystals (54 mg, 95%).
¹H NMR ((CD₃)₂SO, 400 MHz) δ: 1.35 (d, 3H), 3.80 (dd, 3H), 3.92 (m, 1H), 4.35 (dd, 1H), 7.50 (dt, 1H), 7.68 (d, 1H), 7.78 (dd, 1H), 7.90 (d, 1H), 7.98 (dd, 1H), 8.12 (s, 4H).

### Example 2

### 6-bromo-1-methyl-1H-benzo[e]indole-3(2H)-carboximidamide hydrochloride

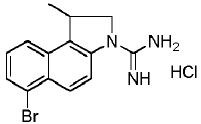

Prepared from 5-bromo-2-naphthoic acid by methods described in Example 1.
¹H NMR ((CD₃)₂SO, 400 MHz) δ: 1.35 (d, 3H), 3.83 (dd, 1H), 3.94 (m, 1H), 4.38 (dd, 1H), 7.48 (t, 1H), 7.79 (d, 1H), 7.82 (d, 1H), 7.94 (d, 1H), 8.11 (d, 1H), 8.21 (s, 4H) .

### Example 3

### 1-Methyl-1H-benzo[e]indole-3(2H)-carboximidamide hydrochloride

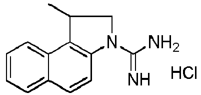

Prepared from tert-butyl (1-bromonaphthalen-2-yl)carbamate by methods described in Example 1.
¹H NMR ((CD₃)₂SO, 400 MHz) δ: 1.36 (d, 3H), 3.81 (dd, 1H), 3.91 (m, 1H), 4.33 (dd, 1H), 7.46 (t, 1H), 7.57 (t, 1H), 7.64 (d, 1H), 7.89 (d, 1H), 7.91 (d, 1H), 7.96 (d, 1H), 8.17 (broad s, 4H).

### Example 4

### 1-methyl-1H-pyrrolo[2,3-c]isoquinoline-3(2H)-carboximidamide hydrochloride

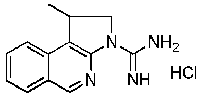

### 1-Methyl-2,3-dihydro-1H-pyrrolo[2,3-c]isoquinoline

Prepared from isoquinolin-3-amine by methods described in Example 1. ¹H NMR (CDCl₃, 400 MHz) δ: 1.44 (d, 3H), 3.35 (dd, 1H), 3.80 (m, 1H), 3.90 (t, 1H), 7.20 (ddd, 1H), 7.51 (ddd, 1H), 7.60 (dd, 1H), 7.80 (d, 1H), 8.73 (s, 1H).

### Step 1: Benzyl ((((benzyloxy)carbonyl)amino)(1-methyl-1H-pyrrolo[2,3-c]isoquinolin-3(2H)-yl)methylene)carbamate

Benzyl ((((benzyloxy)carbonyl)amino)(1H-pyrazol-1-yl)methylene)-carbamate (945 mg, 2.50 mmol) was added to a solution of 1-methyl-2,3-dihydro-1H-pyrrolo[2,3-c]isoquinoline (460 mg, 2.50 mmol)in THF (15 mL). The reaction mixture was stirred at room temperature for 24 h, then at 65°C for 48 h, cooled to room temperature, and was then concentrated at reduced pressure. The residue was purified by silica column chromatography (heptane-EtOAc, 3:2) to give a material (850 mg) still contaminated by the pyrazole reagent. The material was dissolved in EtOAc (50 mL), washed with water (2 x 25 mL) and the organic phase was dried (Na₂SO₄) and concentrated at reduced pressure. The residue was recrystallized from heptane-EtOAc (1:1) to give the title compound as grey crystals (550 mg, 44%).
¹H NMR (CDCl₃, 400 MHz) δ: 1.45 (d, 3H), 3.79 (m, 1H), 4.08 (dd, 1H), 4.36 (dd, 1H), 5.20 (s, 2H), 5.22 (s, 2H), 7.28-7.50 (m, 11H), 7.70 (t, 1H), 7.76 (d, 1H), 7.94 (d, 1H), 8.84 (s, 1H), 12.28 (s, 1H).

### Step 2: 1-Methyl-1H-pyrrolo[2,3-c]isoquinoline-3(2H)-carboximidamide hydrochloride

A solution of benzyl ((((benzyloxy)carbonyl)amino)(1-methyl-1H-pyrrolo[2,3-c]isoquinolin-3(2H)-yl)methylene)carbamate (240 mg, 4.85 mmol) in THF (30 mL) was hydrogenated by stirring for 48 h at room temperature at 1 atm H₂ (g) with Pd/C (220 mg, 10 %) as catalyst. After filtration of the reaction mixture, the filtrate was acidified by addition of 1 M HCl/EtOAc and was then concentrated at reduced pressure. The residue was purified by silica column chromatography (CH₂Cl₂-MeOH-AcOH, 7:1:0.1) to give the title compound as greyish crystals (36 mg, 28%).
¹H NMR ((CD₃)₂SO, 400 MHz) δ: 1.43 (d, 3H), 3.86 (dd, 1H), 4.06 (m, 1H), 4.35 (t, 1H), 7.61 (t, 1H), 7.84 (t, 1H), 7.99 (d, 1H), 8.21 (d, 1H), 8.49 (broad s, 4H), 9.12 (s, 1H).

### Example 5

### 1-Methyl-1H-pyrrolo[2,3-c]quinoline-3(2H)-carboximidamide hydrochloride

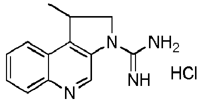

Prepared from quinolin-3-amine by methods described in Example 4.
¹H NMR ((CD₃)₂SO, 400 MHz) δ: 1.39 (d, 3H), 3.84 (d, 1H), 4.00 (m, 1H), 4.37 (t, 1H), 7.67 (m, 2H), 8.01 (m, 2H), 8.62 (broad s, 4H), 9.07 (s, 1H) .

### Example 6

### N,1-Dimethyl-1H-benzo[e]indole-3(2H)-carboximidamide hydrochloride

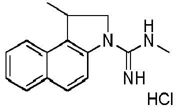

### tert-Butyl (((tert-butoxycarbonyl)imino)(1-methyl-1H-benzo[e]indol-3(2H)-yl)methyl)carbamate

Prepared from tert-butyl (1-bromonaphthalen-2-yl)carbamate by methods described in Example 1.
¹H NMR (CDCl₃, 400 MHz) δ: 1.41 (s, 9H), 1.46 (d, 3H), 1.56 (s, 9H), 3.75 (m, 1H), 4.03 (d, 1H), 4.44 (dd, 1H), 7.63-7.69 (m, 2H), 7.39, (ddd, 1H), 7.49 (ddd, 1H), 7.79 (d, 1H), 7.82 (d, 1H), 10.41 (s, 1H).

### Step 1: tert-Butyl (((tert-butoxycarbonyl)imino)(1-methyl-1H-benzo[e]indol-3(2H)-yl)methyl)(methyl)carbamate

A solution of tert-butyl (((tert-butoxycarbonyl)imino)(1-methyl-1H-benzo[e]indol-3(2H)-yl)methyl)carbamate (80 mg, 0.19 mmol), PPh₃ (76 mg, 0.29 mmol, diisopropyl azodicarboxylate (58 mg, 0.29 mmol), and MeOH (9 mg, 29 mmol) in THF (1 mL) was stirred at room temperature overnight. The reaction mixture was then concentrated at reduced pressure and the residue was purified by silica column chromatography (heptane-EtOAc, 4:1) to give the title compound as an oil (68 mg, 82%). ¹H NMR (CDCl₃, 400 MHz) δ: 1.33, 1.36 (2 broad s, rotamers, 9H), 1.45 (m, rotamers, 3H), 1.55 (s, 9H), 3.13, 3.16 (2s, rotamers, 3H), 3.82 (m, 1H), 3.91 (d, 1H), 4.34 (t, 1H), ca 7.30-7.60 (very broad, 1H), 7.40 (m, 1H), 7.51 (m, 1H), 7.72 (broad d, 1H), 7.80 (d, 1H), 7.83 (d, 1H) .

### Step 2: N,1-Dimethyl-1H-benzo[e]indole-3(2H)-carboximidamide hydrochloride

Prepared as greyish crystals (43 mg, 98%) by the method described in Example 1 (step 9).
¹H NMR ((CD₃)₂SO, 400 MHz) δ: 1.34 (d, 3H), 2.94 (s, 3H), 3.78 (dd, 1H), 3.88 (m, 1H), 4.37 (dd, 1H), 7.44 (t, 1H), 7.57 (t, 1H), 7.58 (d, 1H), 7.88 (m, 2H), 7.95 (d, 1H), 8.36 (broad s, 3H).

### Example 7

### N-Butyl-1-methyl-1H-benzo[e]indole-3(2H)-carboximidamide hydrochloride

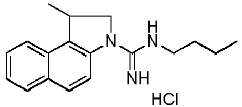

Prepared by methods described in Example 6.
¹H NMR ((CD₃)₂SO, 400 MHz) δ: 0.94 (t, 3H), 1.34 (d, 3H), 1.40 (m, 2H), 1.62 (m, 2H), 3.29 (m, 1H), 3.37 (m, 1H), 3.78 (d, 1H), 3.87 (m, 1H), 4.42 (t, 1H), 7.44 (t, 1H), 7.52-7.59 (m, 2H), 7.88 (t, 2H), 7.94 (d, 1H), 8.37 (s, 2H), 8.53 (t, 1H).

### Example 8

### N-Benzyl-1-methyl-1H-benzo[e]indole-3(2H)-carboximidamide hydrochloride

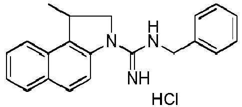

Prepared by methods described in Example 6.
¹H NMR ((CD₃)₂SO, 400 MHz) δ: 1.35 (d, 3H), 3.84 (dd, 1H), 3.89 (m, 1H), 4.45 (dd, 1H), 4.53 (dd, 1H), 4.71 (dd, 1H), 7.35 (tt, 1H), 7.41-7.7.50 (m, 5H), 7.55 (d, 1H), 7.57 (m, 1H), 7.88 (d, 2H), 7.94 (d, 1H), 8.52 (s, 2H), 9.04 (t, 1H).

### Example 9

### N-(4-Chlorobenzyl)-1-methyl-1H-benzo[e]indole-3(2H)-carboximidamide hydrochloride

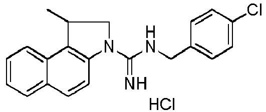

Prepared by methods described in Example 6.
¹H NMR ((CD₃)₂SO, 400 MHz) δ: 1.35 (d, 3H), 3.84 (dd, 1H), 3.89 (m, 1H), 4.45 (dd, 1H), 4.53 (dd, 1H), 4.71 (dd, 1H), 7.42-7.7.59 (m, 7H), 7.88 (d, 2H), 7.94 (d, 1H), 8.54 (s, 2H), 9.07 (t, 1H).

### Example 10

### N-(3,4-Difluorobenzyl)-1-methyl-1H-benzo[e]indole-3(2H)-carboximidamide hydrochloride

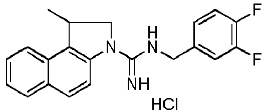

Prepared by methods described in Example 6.
¹H NMR ((CD₃)₂SO, 400 MHz) δ: 1.35 (d, 3H), 3.83-3.94 (m, 2H), 4.44 (dd, 1H), 4.54 (dd, 1H), 4.69 (dd, 1H), 7.34 (m, 1H), 7.42-7.7.65 (m, 5H), 7.88 (d, 2H), 7.94 (d, 1H), 8.54 (s, 2H), 9.04 (t, 1H).

### Example 11

5-HT_{2B} receptor binding (performed at Eurofins Panlabs Taiwan, Ltd.) to human receptor protein expressed in CHO-K1 cells was determined in a ligand displacement assay using ³H-lysergic acid diethylamide (LSD) as radioligand and ketanserine as a standard reference compound. Data is presented as % displacement of radioligand at indicated compound concentrations, see Table 1.

**Table 1.**

| | 5-HT_{2B} receptor binding (% displacement) | | |
|---|---|---|---|
| Compound (Ex. No) | 1 nM | 10 nM | 100 nM |
| 1 | 9 | 49 | 88 |
| 2 | 72 | 96 | 98 |
| 3 | | 63 | 90 |
| 4 | -1 | 16 | 64 |
| 5 | 11 | 28 | 71 |
| 6 | 18 | 57 | 93 |
| 7 | 32 | 77 | 96 |
| 8 | 30 | 80 | 99 |
| 9 | 26 | 55 | 95 |
| 10 | 7 | 50 | 91 |

### Example 12

5-HT_{2B} receptor antagonism (performed at Eurofins Panlabs Taiwan, Ltd.) was determined in CHO-K1 cells expressing human receptor protein as inhibition of 5-HT (5 nM) stimulated IP-1 accumulation measured by HTRF quantitation. SB 206553 was used as a standard reference compound. The antagonistic response is expressed as % inhibition of the 5-HT induced effect.

### Test compound: 1-Methyl-1H-benzo[e]indole-3(2H)-carboximidamide hydrochloride (Example 3)

Results (% inhibition): 2% at 1 nM, 50% at 10 nM, and 100% at 100 nM. The results demonstrated potent antagonistic effects in accordance with receptor binding potencies.

### Example 13

5-HT_{2B} receptor agonism (performed at Eurofins Panlabs Taiwan, Ltd.) was determined in the same model as described above in Example 12 without prior stimulation with 5-HT. The agonistic response is expressed as % of the IP-1 accumulation induced by 5-HT (1 uM) stimulation.

### Test compound: 1-Methyl-1H-benzo[e]indole-3(2H)-carboximidamide hydrochloride (Example 3)

Results (% agonism): -3% at 1 nM, 0% at 10 nM, and -3% at 100 nM.
The results demonstrated the absence of agonistic effects.

## Claims

1. A compound of the general formula I 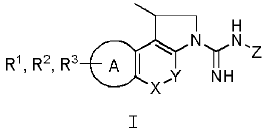 wherein
ring A 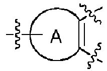 represents a 5-6 membered aromatic or heteroaromatic ring containing 0-3 heteroatoms independently selected from N, O, and S;
R¹, R², and R³, are independently selected from hydrogen, methyl, ethyl, n-propyl, iso-propyl, cyclopropyl, tert-Bu, ethynyl, CF₃, hydroxy, methoxy, ethoxy, iso-propoxy, OCF₃, SCH₃, S(O)₂OH, S(O)CH₃, S(O)₂CH₃, S(O)₂NH₂, S(O)₂N(CH₃)₂, NH₂, NHCH₃, N(CH₃)₂, NHC(O)CH₃, C(O)N(CH₃)₂, F, Cl, Br, I, CN, and 5-6 membered aromatic or heteroaromatic rings containing 0-3 heteroatoms independently selected from N, O, and S;
X-Y, in which X is connected to Y by a single or a double bond or is non-existing, is selected from CH=CH, C(CH₃)=CH, C(F)=CH, C(Cl)=CH, C(OMe)=CH, CH₂-CH₂, N=CH, CH=N, N=N, O-CH₂, O-C(O), NH, NCH₃, O, or S;
Z is selected from hydrogen, methyl, ethyl, n-propyl, iso-propyl, n-butyl, iso-butyl, neo-butyl, tert-butyl, allyl, 2-propynyl, cyclopropyl, cyclopropylmethyl, cyclopentyl, cyclohexyl, CH₂CCH, CF₃, CH₂CF₃, CH₂CH₂F, CH₂CH₂CF₂CF₃, phenyl, benzyl, hydroxy, and methoxy, wherein said phenyl and benzyl groups are optionally mono- or di-substituted by substituents independently selected from methyl, methoxy, F, Cl, and CF₃; and
pharmaceutically acceptable salts, prodrugs, tautomers, and stereoisomers thereof.

2. A compound according to claim 1, wherein 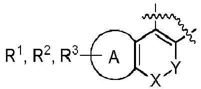 is selected from the group 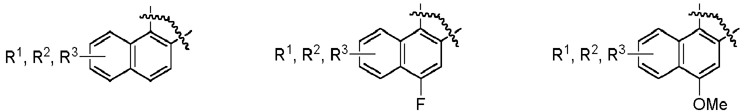 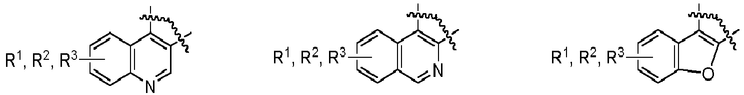 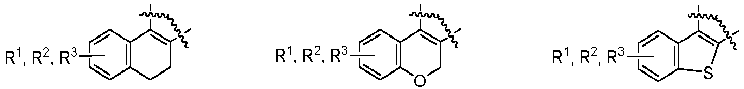 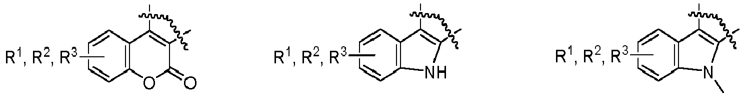 wherein
R¹, R², and R³, are independently selected from hydrogen, methyl, ethyl, n-propyl, iso-propyl, cyclopropyl, tert-Bu, ethynyl, CF₃, hydroxy, methoxy, ethoxy, iso-propoxy, OCF₃, SCH₃, S(O)₂OH, S(O)CH₃, S(O)₂CH₃, S(O)₂NH₂, S(O)₂N(CH₃)₂, NH₂, NHCH₃, N(CH₃)₂, NHC(O)CH₃, C(O)N(CH₃)₂, F, Cl, Br, I, CN, and 5-6 membered aromatic or heteroaromatic rings containing 0-3 heteroatoms independently selected from N, O, and S.

3. A compound according to claim 1 or 2, wherein 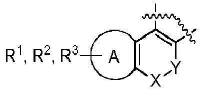 is selected from the group 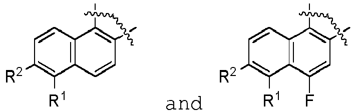 wherein
R¹ is selected from hydrogen, methyl, ethyl, iso-propyl, cyclopropyl, tert-Bu, ethynyl, CF₃, methoxy, OCF₃, SCH₃, S(O)₂NH₂, S(O)₂N(CH₃)₂, F, Cl, Br, I, CN, phenyl, thiophen-2-yl, thiophen-3-yl, pyrazol-3-yl, pyrazol-4-yl, imidazole-2-yl, imidazole-4-yl, isoxazole-3-yl, isoxazole-4-yl; and
R² is selected from hydrogen and F.

4. A compound according to any one of claims 1 to 3,
wherein
Z is selected from hydrogen, methyl, ethyl, n-propyl, iso-propyl, n-butyl, cyclopropyl, allyl, 2-propynyl, CH₂CH₂F, CH₂CF₃, CH₂CH₂CF₂CF₃, phenyl, and benzyl, wherein said phenyl and benzyl groups are optionally mono- or di-substituted by substituents independently selected from methyl, methoxy, F, Cl, and CF₃.

5. A compound according to any one of claims 1 to 4,
wherein 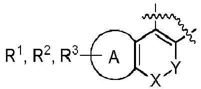 is selected from the group 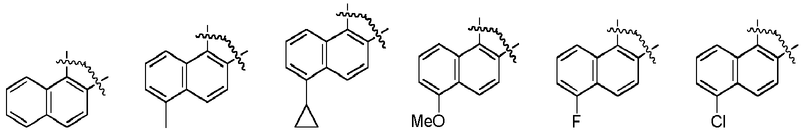 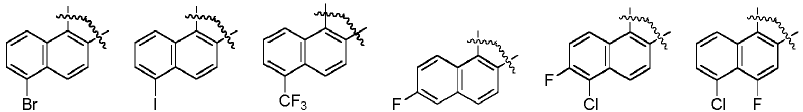 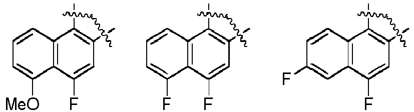 ; and
Z is selected from hydrogen, methyl, n-butyl, cyclopropyl, allyl, 2-propynyl, CH₂CH₂F, CH₂CF₃, CH₂CH₂CF₂CF₃, phenyl, benzyl, wherein said phenyl and benzyl groups are optionally mono- or di-substituted by substituents independently selected from methyl, methoxy, F, Cl, and CF₃.

6. A compound according to claim 1,
wherein
ring A 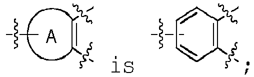 R¹, R², and R³, are independently selected from hydrogen, F, and Br;
X-Y is selected from CH=CH, N=CH, and CH=N; and
Z is selected from hydrogen, methyl, n-butyl, and benzyl, wherein said benzyl is optionally mono- or di-substituted by substituents independently selected from F and Cl.

7. A compound according to any one of claims 1 to 6,
wherein
the absolute configuration is (S) as shown in formula Ia

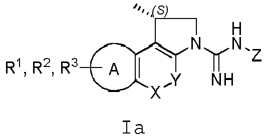

8. A compound according to claim 1, said compound being selected from:
1-Methyl-1H-benzo[e]indole-3(2H)-carboximidamide;
N,1-Dimethyl-1H-benzo[e]indole-3(2H)-carboximidamide;
N-Butyl-1-methyl-1H-benzo[e]indole-3(2H)-carboximidamide;
N-Allyl-1-methyl-1,2-dihydro-3H-benzo[e]indole-3-carboximidamide;
1-Methyl-N-(prop-2-yn-1-yl)-1,2-dihydro-3H-benzo[e]indole-3-carboximidamide;
N-(2-Fluoroethyl)-1-methyl-1,2-dihydro-3H-benzo[e]indole-3-carboximidamide;
N-Benzyl-1-methyl-1H-benzo[e]indole-3(2H)-carboximidamide N-(4-Fluorobenzyl)-1-methyl-1,2-dihydro-3H-benzo[e]indole-3-carboximidamide;
N-(3,4-Difluorobenzyl)-1-methyl-1H-benzo[e]indole-3(2H)-carboximidamide;
N-(4-Chlorobenzyl)-1-methyl-1H-benzo[e]indole-3(2H)-carboximidamide;
1-Methyl-N-(4-(trifluoromethyl)benzyl)-1,2-dihydro-3H-benzo[e]indole-3-carboximidamide;
N-(4-Methoxybenzyl)-1-methyl-1,2-dihydro-3H-benzo[e]indole-3-carboximidamide;
1-Methyl-N-phenyl-1,2-dihydro-3H-benzo[e]indole-3-carboximidamide;
N-(4-Fluorophenyl)-1-methyl-1,2-dihydro-3H-benzo[e]indole-3-carboximidamide;
N-(4-Chlorophenyl)-1-methyl-1,2-dihydro-3H-benzo[e]indole-3-carboximidamide;
1-Methyl-N-(4-(trifluoromethyl)phenyl)-1,2-dihydro-3H-benzo[e]indole-3-carboximidamide;
N-(4-Methoxyphenyl)-1-methyl-1,2-dihydro-3H-benzo[e]indole-3-carboximidamide;
6-Fluoro-1-methyl-1,2-dihydro-3H-benzo[e]indole-3-carboximidamide;
6-Chloro-1-methyl-1,2-dihydro-3H-benzo[e]indole-3-carboximidamide;
6-Chloro-N,1-dimethyl-1,2-dihydro-3H-benzo[e]indole-3-carboximidamide;
N-Butyl-6-chloro-1-methyl-1,2-dihydro-3H-benzo[e]indole-3-carboximidamide;
N-Allyl-6-chloro-1-methyl-1,2-dihydro-3H-benzo[e]indole-3-carboximidamide;
6-Chloro-1-methyl-N-(prop-2-yn-1-yl)-1,2-dihydro-3H-benzo[e]indole-3-carboximidamide;
6-Chloro-N-(2-fluoroethyl)-1-methyl-1,2-dihydro-3H-benzo[e]indole-3-carboximidamide;
N-Benzyl-6-chloro-1-methyl-1,2-dihydro-3H-benzo[e]indole-3-carboximidamide;
6-Chloro-1-methyl-N-phenyl-1,2-dihydro-3H-benzo[e]indole-3-carboximidamide;
6-Bromo-1-methyl-1H-benzo[e]indole-3(2H)-carboximidamide;
6-Bromo-N,1-dimethyl-1,2-dihydro-3H-benzo[e]indole-3-carboximidamide;
6-Bromo-N-butyl-1-methyl-1,2-dihydro-3H-benzo[e]indole-3-carboximidamide;
N-Allyl-6-bromo-1-methyl-1,2-dihydro-3H-benzo[e]indole-3-carboximidamide;
6-Bromo-1-methyl-N-(prop-2-yn-1-yl)-1,2-dihydro-3H-benzo[e]indole-3-carboximidamide;
6-Bromo-N-(2-fluoroethyl)-1-methyl-1,2-dihydro-3H-benzo[e]indole-3-carboximidamide;
N-Benzyl-6-bromo-1-methyl-1,2-dihydro-3H-benzo[e]indole-3-carboximidamide;
6-Bromo-1-methyl-N-phenyl-1,2-dihydro-3H-benzo[e]indole-3-carboximidamide;
6-Iodo-1-methyl-1,2-dihydro-3H-benzo[e]indole-3-carboximidamide;
1,6-Dimethyl-1,2-dihydro-3H-benzo[e]indole-3-carboximidamide;
6-Cyclopropyl-1-methyl-1,2-dihydro-3H-benzo[e]indole-3-carboximidamide;
1-Methyl-6-(trifluoromethyl)-1,2-dihydro-3H-benzo[e]indole-3-carboximidamide;
N,1-Dimethyl-6-(trifluoromethyl)-1,2-dihydro-3H-benzo[e]indole-3-carboximidamide;
N-Butyl-1-methyl-6-(trifluoromethyl)-1,2-dihydro-3H-benzo[e]indole-3-carboximidamide;
N-Allyl-1-methyl-6-(trifluoromethyl)-1,2-dihydro-3H-benzo[e]indole-3-carboximidamide;
1-Methyl-N-(prop-2-yn-1-yl)-6-(trifluoromethyl)-1,2-dihydro-3H-benzo[e]indole-3-carboximidamide;
N-(2-Fluoroethyl)-1-methyl-6-(trifluoromethyl)-1,2-dihydro-3H-benzo[e]indole-3-carboximidamide;
N-Benzyl-1-methyl-6-(trifluoromethyl)-1,2-dihydro-3H-benzo[e]indole-3-carboximidamide;
1-Methyl-N-phenyl-6-(trifluoromethyl)-1,2-dihydro-3H-benzo[e]indole-3-carboximidamide;
6-Methoxy-1-methyl-1,2-dihydro-3H-benzo[e]indole-3-carboximidamide;
6-Chloro-7-fluoro-1-methyl-1,2-dihydro-3H-benzo[e]indole-3-carboximidamide;
6-Chloro-5-fluoro-1-methyl-1,2-dihydro-3H-benzo[e]indole-3-carboximidamide;
5-Fluoro-6-methoxy-1-methyl-1,2-dihydro-3H-benzo[e]indole-3-carboximidamide;
5,6-Difluoro-1-methyl-1,2-dihydro-3H-benzo[e]indole-3-carboximidamide;
7-Fluoro-1-methyl-1H-benzo[e]indole-3(2H)-carboximidamide;
7-Fluoro-N,1-dimethyl-1,2-dihydro-3H-benzo[e]indole-3-carboximidamide;
N-Butyl-7-fluoro-1-methyl-1,2-dihydro-3H-benzo[e]indole-3-carboximidamide;
N-Allyl-7-fluoro-1-methyl-1,2-dihydro-3H-benzo[e]indole-3-carboximidamide;
7-Fluoro-1-methyl-N-(prop-2-yn-1-yl)-1,2-dihydro-3H-benzo[e]indole-3-carboximidamide;
7-Fluoro-N-(2-fluoroethyl)-1-methyl-1,2-dihydro-3H-benzo[e]indole-3-carboximidamide;
N-Benzyl-7-fluoro-1-methyl-1,2-dihydro-3H-benzo[e]indole-3-carboximidamide;
7-Fluoro-1-methyl-N-phenyl-1,2-dihydro-3H-benzo[e]indole-3-carboximidamide;
5,7-Difluoro-1-methyl-1,2-dihydro-3H-benzo[e]indole-3-carboximidamide;
1-Methyl-1H-pyrrolo[2,3-c]isoquinoline-3(2H)-carboximidamide; and
1-Methyl-1H-pyrrolo[2,3-c]quinoline-3(2H)-carboximidamide.

9. A compound according to claim 1, said compound being selected from:
7-Fluoro-1-methyl-1H-benzo[e]indole-3(2H)-carboximidamide;
6-Bromo-1-methyl-1H-benzo[e]indole-3(2H)-carboximidamide;
1-Methyl-1H-benzo[e]indole-3(2H)-carboximidamide;
1-Methyl-1H-pyrrolo[2,3-c]isoquinoline-3(2H)-carboximidamide;
1-Methyl-1H-pyrrolo[2,3-c]quinoline-3(2H)-carboximidamide;
N,1-Dimethyl-1H-benzo[e]indole-3(2H)-carboximidamide;
N-Butyl-1-methyl-1H-benzo[e]indole-3(2H)-carboximidamide;
N-Benzyl-1-methyl-1H-benzo[e]indole-3(2H)-carboximidamide;
N-(4-Chlorobenzyl)-1-methyl-1H-benzo[e]indole-3(2H)-carboximidamide; and
N-(3,4-Difluorobenzyl)-1-methyl-1H-benzo[e]indole-3(2H)-carboximidamide.

10. A compound according to any one of claims 1 to 9 for use as a medicament.

11. A compound according to any one of claims 1 to 9, for use in treatment of fibrosis, cardiovascular diseases, pain, IBD, inflammatory diseases, or cancer.

12. A compound according to any one of claims 1 to 9, for use in treatment of fibrosis, wherein the fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring.

13. A compound according to any one of claims 1 to 9, for use in treatment of a cardiovascular disease, selected from atherosclerosis and hypertension.

14. A compound according to any one of claims 1 to 9, for use in treatment of pain, selected from migraine and pain associated with inflammatory diseases.

15. A compound according to any one of claims 1 to 9, for use in treatment of IBD, selected from Crohn's disease and ulcerous colitis.

16. A compound according to any one of claims 1 to 9, for use in treatment of inflammatory joint diseases including rheumatoid arthritis (RA) and osteoarthritis (OA).

17. A compound according to any one of claims 1 to 9, for use in treatment of cancer including extracellular matrix producing cancers such as breast cancer, pancreas cancer, and liver cancer.

18. Use of a compound according to any one of claims 1 to 9 in the manufacture of a medicament useful in treatment of fibrosis, cardiovascular diseases, pain, IBD, inflammatory diseases, or cancer.

19. Use of a compound according to any one of claims 1 to 9 in the manufacture of a medicament useful in treatment of fibrosis, wherein the fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring.

20. Use of a compound according to any one of claims 1 to 9 in the manufacture of a medicament useful in treatment of cardiovascular disease, selected from atherosclerosis and hypertension.

21. Use of a compound according to any one of claims 1 to 9 in the manufacture of a medicament useful in treatment of pain, selected from migraine and pain associated with inflammatory diseases.

22. Use of a compound according to any one of claims 1 to 9 in the manufacture of a medicament useful in treatment of IBD, selected from Crohn's disease and ulcerous colitis.

23. Use of a compound according to any one of claims 1 to 9 in the manufacture of a medicament useful in treatment of inflammatory joint diseases including rheumatoid arthritis (RA) and osteoarthritis (OA).

24. Use of a compound according to any one of claims 1 to 9 in the manufacture of a medicament useful in treatment of cancer including extracellular matrix producing cancers such as breast cancer, pancreas cancer, and liver cancer.

25. A method of treating fibrosis, cardiovascular diseases, pain, IBD, inflammatory diseases, or cancer comprising administering a therapeutically effective amount of a compound according to any one of claims 1 to 9 to a patient in need thereof.

26. A method of treating fibrosis, wherein the fibrosis is selected from systemic sclerosis, skin fibrosis, liver fibrosis, heart fibrosis, kidney fibrosis, intestinal fibrosis, lung fibrosis including idiopathic pulmonary fibrosis (IPF) and fibrosis associated with pulmonary arterial hypertension (PAH), and fibrosis associated with transplantation, surgery, stenosis, or keloid scarring, comprising administering a therapeutically effective amount of a compound according to any one of claims 1 to 9 to a patient in need thereof.

27. A method of treating cardiovascular diseases, selected from atherosclerosis and hypertension, comprising administering a therapeutically effective amount of a compound according to any one of claims 1 to 9 to a patient in need thereof.

28. A method of treating pain, selected from migraine and pain associated with inflammatory diseases, comprising administering a therapeutically effective amount of a compound according to any one of claims 1 to 9 to a patient in need thereof.

29. A method of treating IBD, selected from Crohn's disease and ulcerous colitis, comprising administering a therapeutically effective amount of a compound according to any one of claims 1 to 9 to a patient in need thereof.

30. A method of treating inflammatory joint diseases including rheumatoid arthritis (RA) and osteoarthritis (OA), comprising administering a therapeutically effective amount of a compound according to any one of claims 1 to 9 to a patient in need thereof.

31. A method of treating cancer including extracellular matrix producing cancers such as breast cancer, pancreas cancer, and liver cancer, comprising administering a therapeutically effective amount of a compound according to any one of claims 1 to 9 to a patient in need thereof.

32. A pharmaceutical composition comprising a compound according to any one of claims 1 to 9, admixed with one or more pharmaceutically acceptable excipients or carriers.

33. The composition according to claim 32, wherein the excipients are selected from the group comprising filling agents, lubricants, flavours, colourings, sweetenings, buffers, acidifying agents, diluents, and preservatives.

34. The composition according to claim 32 or claim 33, which is formulated to be administered orally, by oral inhalation, intramuscularly, intravenously, intraperitoneally, or subcutaneously, via implants, rectally, intranasally, or transdermally; preferably orally.

35. The composition according to any one of claims 32 to 34, further comprising an additional therapeutic agent.
